(19) Europäisches Patentamt / European Patent Office / Office européen des brevets

(11) **EP 4 621 789 A1**

(12) **EUROPEAN PATENT APPLICATION**

(43) Date of publication:
**24.09.2025 Bulletin 2025/39**

(21) Application number: **25154886.3**

(22) Date of filing: **30.01.2025**

(51) International Patent Classification (IPC):
**G16B 40/20** (2019.01)        **G06N 3/00** (2023.01)
**G16B 15/20** (2019.01)

(52) Cooperative Patent Classification (CPC):
**G16B 40/20; G06N 3/00; G06N 3/044; G06N 3/045;
G06N 3/084; G16B 15/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **18.03.2024 JP 2024041978**

(71) Applicant: **FUJITSU LIMITED
Kawasaki-shi, Kanagawa 211-8588 (JP)**

(72) Inventors:
- **Yoshikawa, Hiyori
Kawasaki-shi, Kanagawa, 211-8588 (JP)**
- **Toma, Mitsunori
Kawasaki-shi, Kanagawa, 211-8588 (JP)**

- **Yamazaki, Kimihiro
Kawasaki-shi, Kanagawa, 211-8588 (JP)**
- **Wada, Yuichiro
Kawasaki-shi, Kanagawa, 211-8588 (JP)**
- **Wada, Mutsuyo
Kawasaki-shi, Kanagawa, 211-8588 (JP)**
- **Waida, Hiroki
Kawasaki-shi, Kanagawa, 211-8588 (JP)**
- **Ishii, Yoshiyuki
Kawasaki-shi, Kanagawa, 211-8588 (JP)**
- **Katoh, Takashi
Kawasaki-shi, Kanagawa, 211-8588 (JP)**
- **Nakagawa, Akira
Kawasaki-shi, Kanagawa, 211-8588 (JP)**

(74) Representative: **Hoffmann Eitle
Patent- und Rechtsanwälte PartmbB
Arabellastraße 30
81925 München (DE)**

(54) **CONTROL PROGRAM, CONTROL METHOD, AND INFORMATION PROCESSING DEVICE**

(57) A control program for causing a computer to execute a process includes, when an intermediate representation of input data input to a learned model is changed, evaluating validity of current output data generated from the intermediate representation by the learned model based on a first indicator correlated with an existence probability of output data in a data distribution of learning data used for learning of the learned model, and changing the intermediate representation based on an evaluation result such that validity of output data generated by the learned model increases.

FIG. 1

**Description**

FIELD

The embodiment discussed herein is related to a computer-readable recording medium storing a control program, a control method, and an information processing device.

BACKGROUND

In related art, a deep learning model that handles sequential information, such as the Transformer model, exhibits high performance in understanding input information and predicting outputs having a structure. There is also known a large-scale learned model having a high representation capability, such as AlphaFold2 that performs protein structure prediction. There is a case in which it is desirable to generate various high-quality outputs without changing the parameters of a learned model by utilizing such representation capability of the learned model.

Citation List

Non-Patent Literature

Vaswani et al.: Attention is All You Need, NeurIPS 2017 (2017) and Jumper, J. et al. Highly accurate protein structure prediction with AlphaFold. Nature 596, 583-589 (2021) are disclosed as related art.

SUMMARY OF INVENTION

TECHNICAL PROBLEMS

However, in related art, when some manipulation is performed on an intermediate representation of a learned model and a change is added to an output, the manipulation range of the intermediate representation corresponding to a valid output is not clear, and it is difficult to determine how to perform the manipulation to obtain a valid output.

SOLUTION TO PROBLEM

According to an aspect of the embodiments, a control program for causing a computer to execute a process includes, when an intermediate representation of input data input to a learned model is changed, evaluating validity of current output data generated from the intermediate representation by the learned model based on a first indicator correlated with an existence probability of output data in a data distribution of learning data used for learning of the learned model, and changing the intermediate representation based on an evaluation result such that validity of output data generated by the learned model increases.

EFFECTS OF INVENTION

According to one aspect of the present disclosure, such an effect is obtained that manipulation of an intermediate representation may be controlled such that a valid output is obtained.

BRIEF DESCRIPTION OF DRAWINGS

FIG. 1 is an explanatory diagram illustrating an exemplary embodiment of a control method according to an embodiment;
FIG. 2 is an explanatory diagram illustrating a system configuration example of an information processing system;
FIG. 3 is a block diagram illustrating a hardware configuration example of a control device;
FIG. 4 is an explanatory diagram illustrating a specific example of input data;
FIG. 5 is an explanatory diagram illustrating a specific example of an intermediate representation;
FIG. 6 is a block diagram illustrating a functional configuration example of the control device;
FIG. 7 is an explanatory diagram illustrating an operation example of the control device; and
FIG. 8 is a flowchart illustrating an example of a control processing procedure of the control device.

DESCRIPTION OF EMBODIMENTS

With reference to the drawings, an embodiment of a control program, a control method, and an information processing device according to the present disclosure will be described in detail below.

(Embodiment)

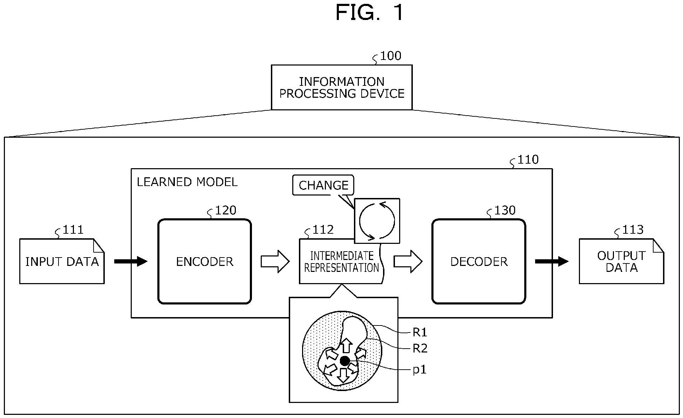

FIG. 1 is an explanatory diagram illustrating an exemplary embodiment of a control method according to the embodiment. In FIG. 1, an information processing device 100 is a computer that controls manipulation of an intermediate representation of input data input to a learned model 110. The learned model 110 is a machine learning model learned by machine learning such as deep learning (learned machine learning model).

For example, the learned model 110 is information in which an algorithm and a learned parameter (weight parameter) are combined. By applying the learned parameter to input data, the learned model 110 derives a result (output data). The learned model 110 converts the input data that has been input into an intermediate representation, and generates output data from the converted intermediate representation.

An intermediate representation is information obtained by extracting a feature from input data. For example, an intermediate representation that is a vector sequence is obtained by extracting a feature from input data that is sequential information. Manipulation of an intermediate representation is manipulation of an intermediate representation converted from input data for obtaining new output data for the input data. For example, an intermediate representation may be manipulated by adding a minute value to the intermediate representation that is a vector sequence and changing the value of the vector sequence.

For example, the learned model 110 is the Transformer model, AlphaFold2, or the like. The Transformer model uses sequential information representing a sentence as input data, and outputs sequential information representing another sentence as output data. AlphaFold2 uses amino acid sequence information as input data, and outputs output data representing a structure (three-dimensional structure) of protein. The Transformer model and AlphaFold2 are large-scale deep learning models having a high representation capability.

For example, Vaswani et al.attention is All You Need, NeurIPS 2017 (2017) and "Radford et al.:Language Models are Unsupervised Multitask Learners, 2019" may be referred to for the Transformer model. For example, Jumper, J. et al.Highly accurate protein structure prediction with AlphaFold.Nature 596, 583-589 (2021) may be referred to for AlphaFold2.

There is a demand for generating a variety of high-quality outputs without changing the parameters of a learned model by utilizing such representation capability of the learned model. For example, there is a case in which it is desirable to list multiple forms that a certain input sequence may take by using AlphaFold2. There is a case in which it is desirable to generate various sentences by using the Transformer model for text generation.

An intermediate representation of a large-scale model abstractly grasps an important feature of data, and may be expected to be suitable for making a meaningful change to an output while maintaining the essence of input data. For this reason, it is conceivable that new output data is generated for input data by manipulating an intermediate representation of a learned model.

However, for example, the Transformer model may not explicitly calculate the probability distribution of an intermediate representation. Consequently, when some manipulation is applied to an intermediate representation and a change is added to an output, the manipulation range of the intermediate representation corresponding to a valid output is not clear based on the data distribution at the time of learning.

In FIG. 1, black circle p1 indicates an intermediate representation (for example, an intermediate representation 112) converted from input data (for example, input data 111). Regions R1 and R2 represent regions that the intermediate representation may take. The region R1 represents a region generalized to some extent through learning. The region R2 is a region including only an intermediate representation corresponding to a valid output. For example, the region R2 corresponds to the distribution of an intermediate representation corresponding to various pieces of input data given to a learning model at the time of learning.

Since the region R1 includes an intermediate representation corresponding to an invalid output, while there is a possibility that a valid output is obtained, there is also a possibility that an invalid output is obtained. Therefore, it is preferable that control is performed such that the intermediate representation is manipulated so as to fit in the region R2 and a valid output is obtained. However, since the region R2 may not be explicitly acquired, it is not clear what kind of manipulation may obtain a valid output.

For example, as a result of manipulating an intermediate representation, the intermediate representation may be moved to a position that deviates from the region R2 and is not on the data distribution of learning data. In this case, the intermediate representation deviates from the range learned by a model, and as a result, there is a possibility that an estimated output with low reliability, which is not included in the learning data, is obtained.

In the present embodiment, description will be given for a control method of controlling manipulation for an intermediate representation of the learned model 110 by using an indicator (first indicator) correlated with an existence probability of output data in the data distribution of learning data used for learning of the learned model 110. Hereinafter, a processing example of the information processing device 100 will be described.

Input data to the learned model 110 is the "input data 111". The learned model 110 includes an encoder 120 and a decoder 130. The encoder 120 converts the input data 111 input to the learned model 110 into the intermediate representation 112. The decoder 130 refers to the intermediate representation 112 and generates output data 113 for the input data 111. For example, in a case where the learned model 110 is "AlphaFold2", the encoder 120 corresponds to Evoformer. The decoder 130 corresponds to Structure module.

The information processing device 100 generates new output data for the input data 111 by changing the intermediate representation 112 (intermediate representation converted from the input data 111 by the encoder 120) of the input data 111 input to the learned model 110. Changing of the intermediate representation 112 corresponds to manipulation of the intermediate representation 112. At this time, for example, the information processing device 100 performs the following processing (1) and (2).

(1) The information processing device 100 evaluates the validity of the current output data 113 generated from the intermediate representation 112 by the learned model 110 based on the first indicator. The first indicator is an indicator correlated with the probability distribution of learning data used for learning of the learned model 110. The current output data 113 corresponds to output data currently focused on.

Learning data is a pair of input data and output data. The probability distribution of learning data represents a distribution state of a probability of, with respect to output data (value taken by a probability variable), taking the value thereof (existence probability). The first indicator is correlated with an existence probability of output data in the data distribution of learning data.

For example, in the case of AlphaFold2, it may be said that output data for which existence probability is high in the data distribution of learning data represents a structure of protein that may exist in the real world. By using the first indicator, the information processing device 100 evaluates the validity of output data such that the validity increases as the structure of protein is more likely to exist in the real world.

In the case of AlphaFold2, for example, a score representing the confidence of an output structure estimated (outputted) for the current output data 113 by the decoder 130 of the learned model 110 may be used as the first indicator. In the case of the Transformer model, for example, a score representing an output probability of a sentence estimated for the current output data 113 by the decoder 130 of the learned model 110 may be used as the first indicator.

For example, the information processing device 100 calculates an energy value corresponding to the current output data 113 by using an energy function including a term defined in a form including the first indicator. With regard to the energy value, for example, the lower the value, the higher the validity of the output data 113. Consequently, the information processing device 100 may perform evaluation of the validity of the current output data 113 in correlation with an existence probability of the output data 113.

(2) The information processing device 100 changes the intermediate representation 112 based on the evaluation result such that the validity of the output data 113 generated by the learned model 110 increases. For example, the information processing device 100 changes the intermediate representation 112 based on the calculated energy value such that the energy function is minimized. For example, changing of the intermediate representation 112 corresponds to searching for the intermediate representation 112 that minimizes the energy function.

Describing in more detail, for example, it is assumed that the energy function is differentiable with respect to the intermediate representation 112. In this case, the information processing device 100 changes the intermediate representation 112 based on a gradient of the energy function using a gradient method. It is assumed that the energy function is not differentiable with respect to the intermediate representation 112 or the gradient method falls into a local solution. In this case, the information processing device 100 changes the intermediate representation 112 by using heuristic search.

When the intermediate representation 112 is changed, the information processing device 100 generates new output data 113 for the input data 111 from the changed intermediate representation 112 by the learned model 110. For example, the information processing device 100 repeatedly executes the above processing (1) and (2) with the generated new output data 113 as the current output data 113, until a predetermined end condition is satisfied.

In this way, according to the information processing device 100, manipulation of the intermediate representation 112 may be controlled such that a valid output (output data 113) is obtained. For example, the information processing device 100 may guide the intermediate representation 112 to fit in the region R2 by limiting the search range of the intermediate representation 112 using the first indicator in manipulation of the intermediate representation 112.

Consequently, the information processing device 100 may add a change to the intermediate representation 112 in a direction in which there is a high possibility that a valid output is obtained, and may obtain a valid output more efficiently than in a case where random manipulation is repeated for the intermediate representation 112.

(System Configuration Example of Information Processing System 200)

Next, a system configuration example of an information processing system 200 including the information processing device 100 illustrated in FIG. 1 will be described. A case in which the information processing device 100 illustrated in FIG. 1 is applied to a control device 201 in the information processing system 200 will be described as an example.

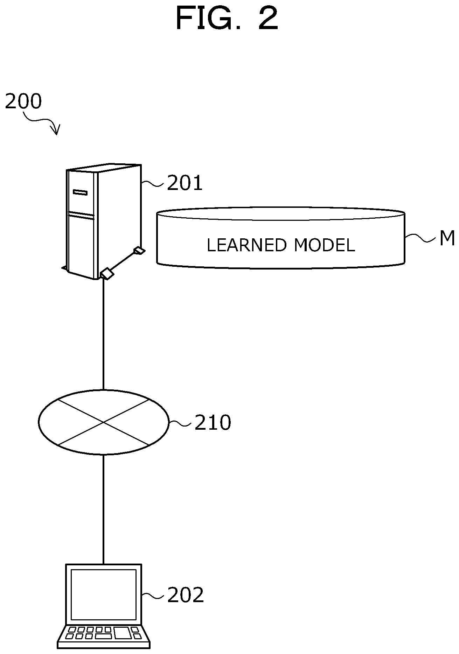

FIG. 2 is an explanatory diagram illustrating a system configuration example of the information processing system 200. In FIG. 2, the information processing system 200 includes the control device 201 and a client device 202. In the information processing system 200, the control device 201 and the client device 202 are coupled to each other via a wired or wireless network 210. For example, the network 210 is the Internet, a local area network (LAN), a wide area network (WAN), or the like.

The control device 201 is a computer that includes a learned model M and controls manipulation of an intermediate representation of input data input to the learned model M. For example, the learned model M is a learned deep learning model such as the Transformer model or AlphaFold2. For example, the control device 201 is a server. For example, the learned model 110 illustrated in FIG. 1 corresponds to the learned model M.

The client device 202 is a computer used by a user of the information processing system 200. For example, a user is a person who predicts the structure of a protein from an amino acid sequence or generates another sentence (a translated sentence or the like) from a certain sentence. For example, the client device 202 is a personal computer (PC), a tablet PC, a smartphone, or the like.

Although the control device 201 and the client device 202 are separately provided, this is not construed in a limiting sense. For example, the control device 201 may be realized by the client device 202. A plurality of client devices 202 may be included in the information processing system 200.

(Hardware Configuration Example of Control Device 201)

Next, a hardware configuration example of the control device 201 will be described.

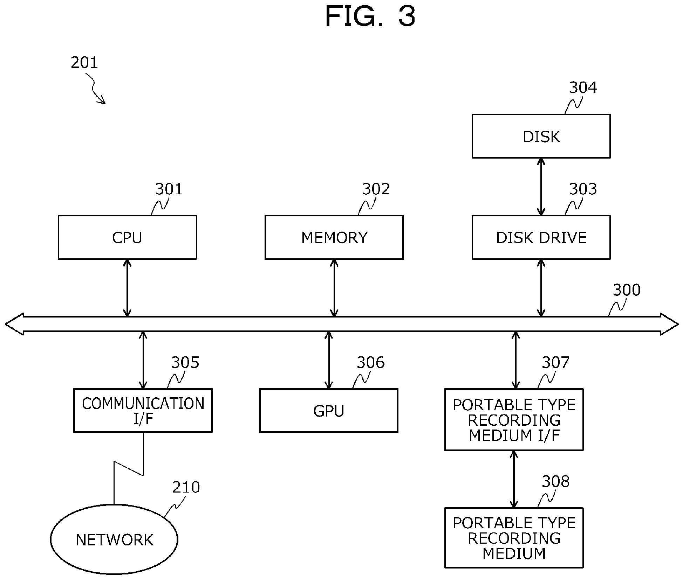

FIG. 3 is a block diagram illustrating a hardware configuration example of the control device 201. In FIG. 3, the control device 201 includes a central processing unit (CPU) 301, a memory 302, a disk drive 303, a disk 304, a communication interface (I/F) 305, a graphics processing unit (GPU) 306, a portable type recording medium I/F 307, and a portable type recording medium 308. These constituent units are coupled to each other by a bus 300.

The CPU 301 takes overall control of the control device 201. The GPU 306 performs arithmetic processing such as image processing and natural language processing. The CPU 301 and the GPU 306 may include a plurality of cores. For example, the memory 302 includes a read-only memory (ROM), a random-access memory (RAM), and the like. A program stored in the memory 302 is loaded into the CPU 301, thereby causing the CPU 301 to execute the coded processing.

The disk drive 303 controls reading and writing of data from and to the disk 304 in accordance with the control of the CPU 301. The disk 304 stores data written under the control of the disk drive 303. For example, the disk 304 is a magnetic disk, an optical disk, or the like.

The communication I/F 305 is coupled to the network 210 via a communication line, and is coupled to an external computer (for example, the client device 202 illustrated in FIG. 2) via the network 210. The communication I/F 305 functions as an interface between the network 210 and the inside of the device, and controls input and output of data from and to the external computer. For example, the communication I/F 305 is a modem, a LAN adapter, or the like.

The portable type recording medium I/F 307 controls reading and writing of data from and to the portable type recording medium 308 in accordance with the control of the CPU 301. The portable type recording medium 308 stores data written under the control of the portable type recording medium I/F 307. For example, the portable type recording medium 308 is a compact disc (CD)-ROM, a Digital Versatile Disk (DVD), a Universal Serial Bus (USB) memory, or the like.

In addition to the above-described constituent units, for example, the control device 201 may include an input device, a display, a printer, a scanner, a microphone, a speaker, and the like. Of the constituent units described above, for example, the control device 201 does not have to include the GPU 306, the portable type recording medium I/F 307, and the portable type recording medium 308.

(Hardware Configuration Example of Client Device 202)

Since a hardware configuration example of the client device 202 is similar to the hardware configuration example of the control device 201 illustrated in FIG. 3 for example, description thereof will be omitted. However, in addition to the constituent units illustrated in FIG. 3, for example, the client device 202 includes an input device, a display, and the like.

(Specific Example of Input Data)

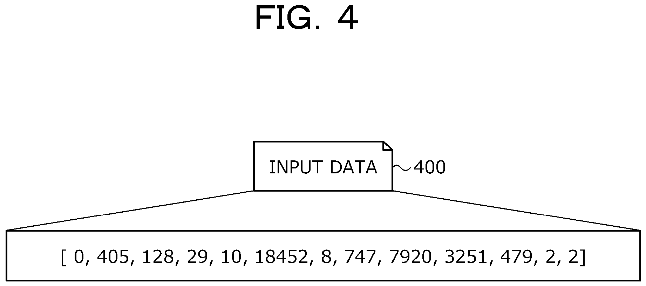

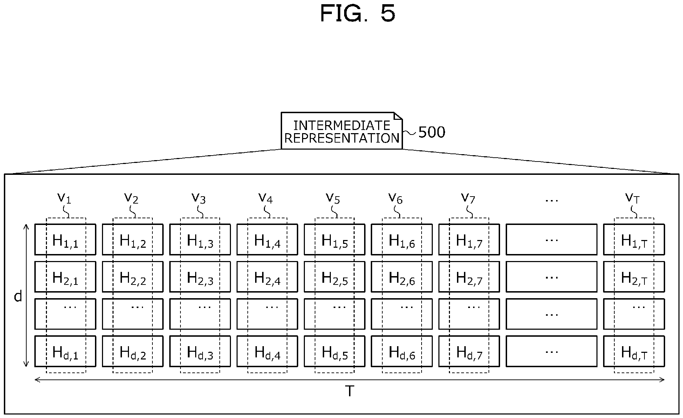

Next, with reference to FIG. 4 and FIG. 5, specific examples of input data to be input to the learned model M illustrated in FIG. 2 and an intermediate representation will be described. A case is assumed in which sequential information representing a sentence (input data) is input with a case where the learned model M is the "Transformer model" as an example.

FIG. 4 is an explanatory diagram illustrating a specific example of input data. In FIG. 4, input data 400 is sequential information indicating a token identifier (ID) string representing a certain sentence. A token corresponds to a sentence (text) divided into units such as words, sub-words, and symbols. A token ID is an identifier for identifying a token.

The input data 400 corresponds to an input text "it'sacharming and often affecting journey." subjected to pre-processing such as Tokenization. For example, the pre-processing may be executed by the control device 201, or may be executed by another computer different from the control device 201 (for example, the client device 202).

FIG. 5 is an explanatory diagram illustrating a specific example of an intermediate representation. In FIG. 5, an intermediate representation 500 is information converted from the input data 400 by extracting a feature from the input data 400 illustrated in FIG. 4. The intermediate representation 500 is a vector sequence in which vectors $v_1$ to $v_T$ corresponding to respective token IDs are arranged for the length T (number of token IDs) of the sequence. Each of the vectors $v_1$ to $v_T$ is a d-dimensional vector.

Although illustration is omitted, for example, in a case where the learned model M is "AlphaFold2", the input data is amino acid sequence information. An intermediate representation is a single representation and a pair representation. The single representation is a vector sequence. The pair representation is sequence information ($T \times T \times d$ dimension) representing the degree of similarity between sequences (between vectors).

(Functional Configuration Example of Control Device 201)

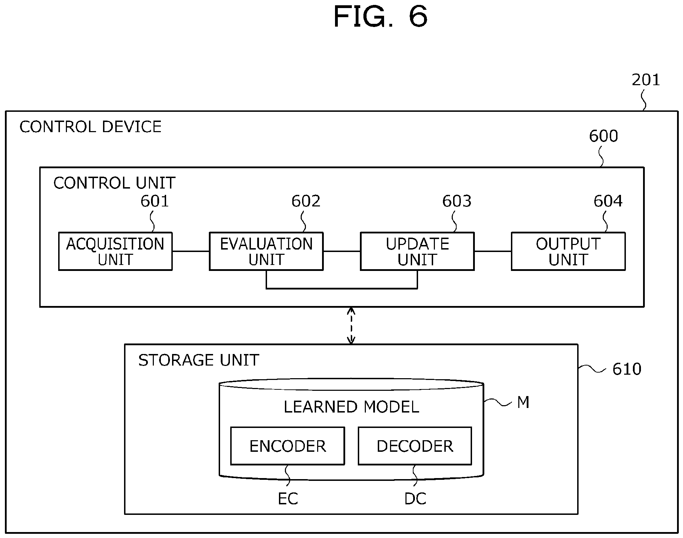

Next, with reference to FIG. 6, a functional configuration example of the control device 201 will be described.

FIG. 6 is a block diagram illustrating a functional configuration example of the control device 201. In FIG. 6, the control device 201 includes an acquisition unit 601, an evaluation unit 602, an update unit 603, an output unit 604, and a storage unit 610. The acquisition unit 601 to the output unit 604 are functions serving as a control unit 600. For example, the functions are realized by causing the CPU 301 to execute a program stored in a storage device such as the memory 302, the disk 304, or the portable type recording medium 308 illustrated in FIG. 3, or by the communication I/F 305 or the GPU 306. For example, a processing result of each functional unit is stored in a storage device such as the memory 302 or the disk 304.

For example, the storage unit 610 is realized by a storage device such as the memory 302 or the disk 304. Although a case where the storage unit 610 is included in the control device 201 will be described, this is not construed in a limiting sense. For example, there may be a case in which the storage unit 610 is included in an external device different from the control device 201, and the storage content of the storage unit 610 may be referred to from the control device 201 via the network 210. The storage unit 610 stores various types of information to be referred to or updated in the processing of each functional unit.

For example, the storage unit 610 stores the learned model M. In the following description, there are cases in which input data to the learned model M is referred to as "input data x", an intermediate representation of the learned model M is referred to as "intermediate representation H", and output data of the learned model M is referred to as "output data y".

The learned model M includes an encoder EC and a decoder DC. For example, the encoder EC converts the input data x input to the learned model M into the intermediate representation H. For example, with reference to the intermediate representation H from the encoder EC, the decoder DC generates output data for the input data x.

The acquisition unit 601 acquires the input data x to the learned model M. For example, the input data x is text sequential information or amino acid sequence information. Text sequential information is sequential information representing a sentence. Amino acid sequence information is sequence information representing the order in which amino acids constituting a protein are arranged. The input data x is information on which pre-processing such as Tokenization has been performed. However, pre-processing such as Tokenization may be performed in the control device 201.

In the case where the learned model M is the "Transformer model", for example, the input data x is the input data 400 illustrated in FIG. 4. For example, the acquisition unit 601 acquires the input data x (for example, the input data 400) by reception from the client device 202 illustrated in FIG. 2. The acquisition unit 601 may acquire the input data x by operation input of a user using an unillustrated input device.

In the following description, there is a case where changing of the intermediate representation H is referred to as manipulation of the intermediate representation H. For example, manipulation of the intermediate representation H is performed by updating the intermediate representation H.

When the intermediate representation H of the input data input to the learned model M is manipulated (changed), the evaluation unit 602 evaluates the validity of the current output data y generated from the intermediate representation H by the learned model M based on the first indicator. The first indicator is an indicator correlated with an existence probability of output data in the data distribution of learning data used for learning of the learned model M. The current output data y corresponds to the output data y currently focused on.

In the case where the learned model M is the "Transformer model", for example, the intermediate representation H is the intermediate representation 500 illustrated in FIG. 5. The control device 201 manipulates the intermediate representation H for obtaining valid new output data y. The initial intermediate representation H to be manipulated is the intermediate representation H obtained by inputting the acquired input data x to the encoder EC of the learned model M. The initial output data y is the output data y obtained by inputting the initial intermediate representation H to the decoder DC of the learned model M.

The second or subsequent intermediate representation H to be manipulated is the intermediate representations H updated by the update unit 603. The second or subsequent output data y is the output data y obtained by inputting the intermediate representation H updated by the update unit 603 to the decoder DC of the learned model M.

The evaluation unit 602 may evaluate the validity of the current output data y based on the first indicator and a second indicator. The second indicator is an indicator representing the closeness to target output data $y^{target}$. For example, in a case where the target output data $y^{target}$ exists, the evaluation unit 602 evaluates the validity of the current output data y by using not only the first indicator but also the second indicator.

For example, the evaluation unit 602 calculates an energy value corresponding to the current output data y by using an energy function E(y) including a first term defined in a form including the first indicator and a second term defined in a form including the second indicator. The energy function E(y) is a function in which an energy value is determined according to the current output data y, and indicates that the smaller the energy value, the higher the validity of the current output data y.

For example, the energy function E(y) may be defined by a single indicator or a combination of a plurality of indicators using the following formula (1) (k = 1, 2, ..., ! = 1, 2, ...).

$$E(y) = \sum_k w_k A_k(y) + \sum_l \lambda_l D_l(y, y^{target}) \quad ...(1)$$

$A_k(y)$ is one indicator representing the validity of the output data y, and is defined in a form including the first indicator. $W_k$ is a weight for $A_k(y)$ and may be arbitrarily set. $\Sigma w_k A_k(y)$ in the above formula (1) corresponds to the first term described above. $D_l(y, y^{target})$ is an indicator representing the closeness to the target output data $y^{target}$ (second indicator) when the target output data $y^{target}$ exists. $Y^{target}$ may be arbitrarily set. As $y^{target}$, for example, a target structure of protein may be set.

The initial output data y (hereinafter referred to as "output data $y_0$") generated from the initial intermediate representation H (hereinafter referred to as "intermediate representation $H_0$") converted from the input data x may be set as $y^{target}$. In this case, it may be said that $D_l(y, y^{target})$ is an indicator for evaluating deviation from the starting point (output data $y_0$). $\lambda_l$ is a weight for D $(y, y^{target})$ and may be arbitrarily set. $\Sigma \lambda_l D_l(y, y^{target})$ in formula (1) corresponds to the second term described above.

$A_k(y)$ and $D_l(y, y^{target})$ may be defined in a form including an error with respect to a true value. For example, $A_k(y)$ may be defined as in the following formula (2). Note that $A_k{\sim}(y)$ corresponds to $A_k(y)$ with an error added thereto. $A_k{\sim}$ represents $A_k$ with ⁻ over it. $\varepsilon_A$ indicates an error. $G_A$ is a constant (maximum value of error).

$$\bar{A}(y) = A(y) + \varepsilon_A, |\varepsilon_A| \le G_A \quad ...(2)$$

$D_l(y, y^{target})$ may be defined as in the following formula (3). Note that $D_l{\sim}(y, y^{target})$ corresponds to $D_l(y, y^{target})$ with an error added thereto. $D_l{\sim}$ represents $D_l$ with ⁻ over it. $\varepsilon_D$ indicates an error. $G_D$ is a constant (maximum value of error).

$$\bar{D}(y, y^{target}) = D(y, y^{target}) + \varepsilon_D, |\varepsilon_D| \le G_D \quad ...(3)$$

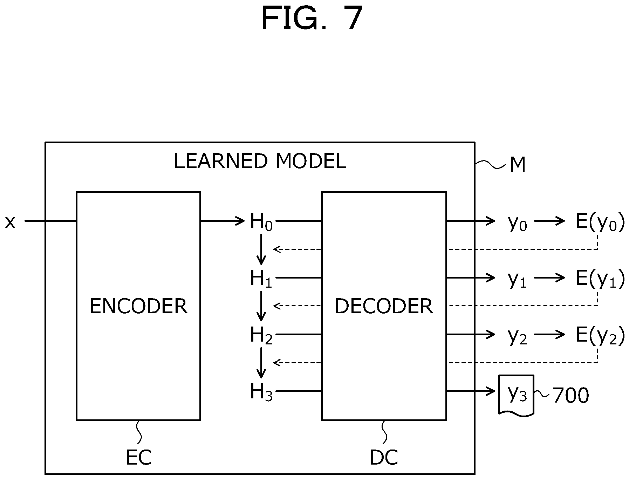

Specific examples of $A_k(y)$ and $D_l(y, y^{target})$ included in the above formula (1) will be described later. A specific processing example of evaluating the validity of the current output data y will be described later with reference to FIG. 7.

The update unit 603 updates (changes) the intermediate representation H based on an evaluation result such that the validity of the output data y generated by the learned model M increases. For example, the update unit 603 updates the intermediate representation H based on the calculated energy value such that the energy function E(y) is minimized. Updating of the intermediate representation H corresponds to searching for the intermediate representation H that minimizes the energy function E(y).

It is assumed that the energy function E(y) is differentiable with respect to the intermediate representation H. In this case, the update unit 603 may update the intermediate representation H using a gradient method (search algorithm) based on a gradient of the energy function E(y). For example, stochastic gradient descent (SGD) may be used as the gradient method.

Describing in more detail, for example, the update unit 603 obtains a gradient of the energy function E(y) based on the calculated energy value. The update unit 603 updates the intermediate representation H based on the gradient of the energy function E(y) and a learning rate. The learning rate may be arbitrarily set.

On the other hand, it is assumed that the energy function E(y) is not differentiable with respect to the intermediate representation H or the gradient method falls into a local solution. In this case, the update unit 603 may update the intermediate representation H using heuristic search (search algorithm) such that the energy value decreases. For example, the Metropolis-Hastings method may be used as the heuristic search.

The update unit 603 generates the output data y (second or subsequent output data y) for the input data x by inputting the updated intermediate representation H to the decoder DC of the learned model M. Consequently, the current output data y is updated. A specific processing example of updating the intermediate representation H and the output data y will be described later with reference to FIG. 7.

The update unit 603 determines whether a predetermined end condition is satisfied as a result of updating the intermediate representation H. For example, the predetermined end condition is a convergence condition for determining that the intermediate representation H has converged (has approached the optimal value), and may be arbitrarily set.

For example, the update unit 603 may set the predetermined end condition to a condition that the update of the intermediate representation H is executed a predetermined number of times. In a case where the intermediate representation H is searched for using the gradient method (SGD), the update unit 603 may set the predetermined end condition to a condition that a change in the gradient of the energy function E(y) is equal to or smaller than a threshold.

When it is determined that the predetermined end condition is not satisfied, the evaluation unit 602 may evaluate the validity of the current output data y. The current output data y to be evaluated is the output data y (second or subsequent output data y) generated from the updated intermediate representation H. Consequently, evaluation of the current output data y and update of the intermediate representation H are repeatedly executed until it is determined that the predetermined end condition is satisfied.

The output unit 604 outputs the output data y for the input data x. For example, when it is determined that the predetermined end condition is satisfied, the output unit 604 outputs the output data y (generation result) generated from the updated intermediate representation H.

The output unit 604 may output the output data y (generation result) generated from the updated intermediate representation H in association with the input data x. Consequently, the output unit 604 makes it easier to specify which input data x the output data y (generation result) corresponds to. The output unit 604 may output the output data y (generation result) generated from the updated intermediate representation H in association with the output data $y_0$ (initial output data y) generated from the intermediate representation $H_0$ (initial intermediate representation H). Consequently, the output unit 604 collectively outputs new output data y (generation result) obtained by manipulating the intermediate representation H together with the initial output data y.

For example, the output form of the output unit 604 is storage into a storage device such as the memory 302 or the disk 304, transmission to another computer by the communication I/F 305, display on an unillustrated display, printing and outputting to an unillustrated printer, or the like.

For example, it is assumed that the input data x to the learned model M is received from the client device 202. In this case, when the predetermined end condition is satisfied as a result of updating the intermediate representation H, the output unit 604 may transmit, to the client device 202, the output data y obtained by inputting the updated intermediate representation H to the decoder DC.

For example, the control device 201 may add a change to the energy function E(y) and manipulate the intermediate representation H for obtaining still another piece of output data y for the input data x. For example, changing of the energy function E(y) may be changing of the combination of indicators defined in the energy function E(y) or changing of weights $w_k$, $\lambda_l$.

There is a case in which the output data y having a structure different from that of the generated output data y (for example, a protein structure or a sentence structure) is desired. In this case, for example, the control device 201 may use $D_l$ (y, $y^{target}$) in the above formula (1) as an indicator for evaluating the deviation from the generated output data y.

For example, the functional units of the control device 201 (the acquisition unit 601 to the output unit 604) may be realized by a plurality of computers in the information processing system 200 (for example, the control device 201 and the client device 202). In this case, for example, exchange between the functional units of the different computers is performed by transmission and reception between the functional units via the network 210.

(Specific Examples of $A_k(y)$ and $D_l(y, y^{target})$)

Next, specific examples of $A_k(y)$ and $D_l(y, y^{target})$ included in the above formula (1) will be described.

• AlphaFold2

First, specific examples of $A_k(y)$ and $D_l(y, y^{target})$ will be described with the case where the learned model M is "AlphaFold2" as an example. AlphaFold2 is a deep learning model that uses amino acid sequence information as the input data x and outputs the output data y representing a structure (three-dimensional structure) of protein.

In this case, as $A_1(y)$ (k = 1), an indicator representing the confidence of an output structure estimated for the current output data y by AlphaFold2 may be used. For example, the confidence is a predicted local distance difference test (pLDDT).

Note that a smaller energy value of the energy function E(y) indicates a higher validity of the current output data y. For this reason, a reciprocal or negative value of the confidence is used for $A_1(y)$. For example, $A_1(y)$ corresponds to the first indicator.

As $A_2(y)$ (k = 2), a score representing the likeness to a target structure of protein estimated for the structure of protein represented by the current output data y by a first classification model different from the learned model M, may be used. The first classification model is a separately learned classification model.

For example, it is assumed that when the default output structure (output data $y_0$) is a close structure, a form of an open structure is desired as a target structure. In this case, for example, the first classification model inputs the output structure (current output data y) and outputs a score representing the likeness to the open structure.

Note that a smaller energy value of the energy function E(y) indicates a higher validity of the current output data y. For this reason, a reciprocal or negative value of a score representing the likeness to a target structure of protein is used for $A_2(y)$. For example, it may be said that $A_2(y)$ is one of indicators representing the closeness to the target output data $y^{target}$, and corresponds to the second indicator. It may be said that $A_2(y)$ is an external indicator since the first classification model different from the learned model M is used.

As $A_3(y)$ (k = 3), the degree of similarity between a known structure similar to the structure of protein represented by the output data $y_0$ among the known structures of proteins stored in a structure database (not illustrated) and the structure of protein represented by the current output data y may be used. The output data $y_0$ is the initial output data y generated from the initial intermediate representation H (intermediate representation $H_0$).

For example, $A_3(y)$ is useful in a case where a user wants to actively search for an unknown structure. For example, it may be said that $A_3(y)$ is one of indicators representing the closeness to the target output data $y^{target}$, and corresponds to the second indicator.

The known structure similar to the structure of protein represented by the output data $y_0$ may be specified by using any existing technique. For example, the control device 201 may specify, as the similar known structure, a structure in which an inter-vector distance (Euclidean distance) from the structure of protein represented by the output data $y_0$ is equal to or smaller than a threshold.

For example, the structure database is stored in the storage unit 610. The structure database may be stored in an external device accessible from the control device 201 via the network 210. In this case, the control device 201 may refer to the structure database by accessing the external device.

As $D_1(y, y^{target})$ (l = 1), the degree of similarity between the electron density distribution corresponding to a target structure of target protein and the electron density distribution corresponding to the structure of protein represented by the current output data y may be used. For example, $D_1(y, y^{target})$ corresponds to the second indicator.

Note that a smaller energy value of the energy function E(y) indicates a higher validity of the current output data y. For this reason, a reciprocal or negative value of the degree of similarity between the electron density distributions is used for $D_1(y, y^{target})$.

For example, $D_1(y, y^{target})$ is used in a case where a specific target structure is not clear but the electron density distribution corresponding to the target structure is known and an atomic structure that approximates the electron density distribution is desired to be estimated. The electron density distribution corresponding to each structure may be specified by using any existing technique. For example, the control device 201 may specify the electron density distribution by performing structural analysis on the structure of protein represented by the current output data y.

• Transformer Model

Next, specific examples of $A_k(y)$ and $D_l(y, y^{target})$ will be described with the case where the learned model M is the "Transformer model" as an example. The Transformer model is a deep learning model that uses sequential information representing a sentence (for example, a token ID string) as input data and outputs sequential information representing another sentence as output data.

In this case, as $A_1(y)$ (k = 1), an indicator representing an appearance probability of a sentence estimated for the current output data y by the Transformer model may be used. For example, an appearance probability of a sentence corresponds to a sentence score indicating the level of validity of an output sentence.

Note that a smaller energy value of the energy function $E(y)$ indicates a higher validity of the current output data y. For this reason, a reciprocal or negative value of an appearance probability of a sentence is used for $A_1(y)$. For example, $A_1(y)$ corresponds to the first indicator.

As $A_2(y)$ (k = 2), a score indicating the degree of certainty of output data in the data distribution of learning data used for learning of the learned model M estimated for the current output data y by a second classification model learned by using the learning data, may be used.

The second classification model is a classification model separately learned using the same learning data as the learned model M. For example, a score estimated (output) by the second classification model corresponds to a score obtained by evaluating the degree of certainty of a sentence from a viewpoint different from the learned model M (Transformer model).

Note that a smaller energy value of the energy function $E(y)$ indicates a higher validity of the current output data y. For this reason, a reciprocal or negative value of a score indicating the degree of certainty of output data is used for $A_2(y)$. For example, $A_2(y)$ corresponds to the first indicator. It may be said that $A_2(y)$ is an external indicator since the second classification model different from the learned model M is used.

As $A_3(y)$ (k = 3), a score representing the degree of conformance to an object label estimated for a sentence represented by the current output data y by a third classification model different from the learned model M, may be used. An object label is a label serving as an object (target) out of a plurality of labels (classes) that classifies sentences.

The third classification model is a separately learned classification model, and is, for example, an emotional polarity classifier. For example, it is assumed that the plurality of labels is "positive", "negative", and the like, and the object label is "positive". In this case, for example, the third classification model inputs the current output data y and outputs a score representing positiveness.

Note that a smaller energy value of the energy function $E(y)$ indicates a higher validity of the current output data y. For this reason, a reciprocal or negative value of a score representing the degree of conformance to an object label is used for $A_3(y)$. For example, it may be said that $A_3(y)$ is one of indicators representing the closeness to the target output data $y^{target}$, and corresponds to the second indicator. It may be said that $A_3(y)$ is an external indicator since the third classification model different from the learned model M is used. For example, $A_3(y)$ is useful in a case where a user wants to generate a sentence of a specific nuance (for example, positive or negative).

As $D_1(y, y^{target})$ (l = 1), an edit distance between an original sentence and a sentence represented by the current output data y may be used. An original sentence is a sentence represented by the initial output data y (output data $y_0$) generated from the initial intermediate representation H (intermediate representation $H_0$) converted from the input data x.

For example, it may be said that $D_1(y, y^{target})$ is an indicator for evaluating a deviation from the original sentence, and corresponds to the second indicator. For example, $D_1(y, y^{target})$ is useful in a case where a user wants to generate a sentence that is not similar to the original sentence (output data $y_0$).

(Operation Example of Control Device 201)

Next, with reference to FIG. 7, an operation example of the control device 201 will be described. An operation example of the control device 201 will be described with the case where the learned model M is "AlphaFold2" as an example. The output data y for the input data x is referred to as "output structure y". For example, the output structure y is coordinate information that specifies a structure (three-dimensional structure) of protein.

FIG. 7 is an explanatory diagram illustrating an operation example of the control device 201. In FIG. 7, first, the control device 201 acquires the input data x to the learned model M. The input data x is amino acid sequence information. The control device 201 acquires the intermediate representation $H_0$ (initial intermediate representation H) converted by inputting the acquired input data x to the encoder EC of the learned model M.

The control device 201 acquires an output structure $y_0$ (initial output structure) generated by inputting the acquired intermediate representation $H_0$ to the decoder DC of the learned model M. Next, the control device 201 acquires various indicators to be used for the energy function $E(y)$ with respect to the generated output structure $y_0$. For example, the various indicators are $A_1(y)$ to $A_3(y)$, $D_1(y, y^{target})$, and the like described above.

The control device 201 calculates an energy value $E(y_0)$ for the output structure $y_0$ based on the acquired various indicators using the above formula (1). Next, the control device 201 updates the intermediate representation $H_0$ to an intermediate representation $H_1$ by a predetermined search algorithm based on the calculated energy value $E(y_0)$ such that the energy function $E(y)$ is minimized.

Next, the control device 201 acquires an output structure $y_1$ generated by inputting the updated intermediate representation $H_1$ to the decoder DC of the learned model M. The control device 201 acquires various indicators to be used for the energy function $E(y)$ with respect to the output structure $y_1$. The control device 201 determines whether a predetermined end condition is satisfied.

A case is assumed in which the predetermined end condition is not satisfied.

In this case, the control device 201 calculates an energy value $E(y_1)$ for the output structure $y_1$ (corresponding to the current output data y) based on the acquired various indicators using the above formula (1). Next, the control device 201 updates the intermediate representation $H_1$ to an intermediate representation $H_2$ by a predetermined search algorithm based on the calculated energy value $E(y_1)$ such that the energy function $E(y)$ is minimized.

Next, the control device 201 acquires an output structure $y_2$ generated by inputting the updated intermediate representation $H_2$ to the decoder DC of the learned model M. The control device 201 acquires various indicators to be used for the energy function $E(y)$ with respect to the output structure $y_2$. The control device 201 determines whether the predetermined end condition is satisfied.

A case is assumed in which the predetermined end condition is not satisfied.

In this case, the control device 201 calculates an energy value $E(y_2)$ for the output structure $y_2$ (corresponding to the current output data y) based on the acquired various indicators using the above formula (1). Next, the control device 201 updates the intermediate representation $H_2$ to an intermediate representation $H_3$ by a predetermined search algorithm based on the calculated energy value $E(y_2)$ such that the energy function $E(y)$ is minimized.

Next, the control device 201 acquires an output structure $y_3$ generated by inputting the updated intermediate representation $H_3$ to the decoder DC of the learned model M. The control device 201 acquires various indicators to be used for the energy function $E(y)$ with respect to the output structure $y_3$. The control device 201 determines whether the predetermined end condition is satisfied.

A case is assumed in which the predetermined end condition is satisfied.

In this case, the control device 201 outputs the output structure $y_3$ generated from the updated intermediate representation $H_3$ as a generation result 700. In this way, the control device 201 may add a change to the intermediate representation H in a direction in which there is a high possibility that a valid structure is obtained, and may efficiently obtain the valid generation result 700 (output structure $y_3$).

(Control Processing Procedure of Control Device 201)

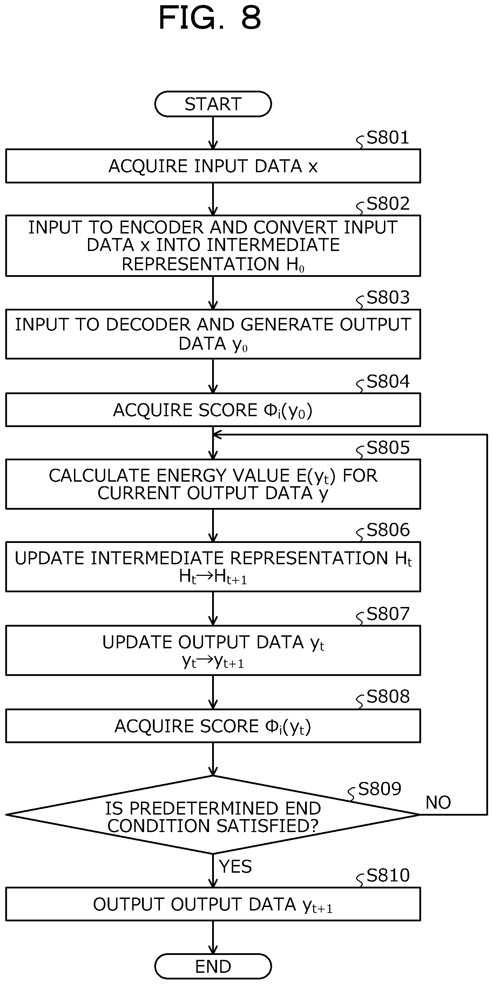

Next, with reference to FIG. 8, a control processing procedure of the control device 201 will be described.

FIG. 8 is a flowchart illustrating an example of a control processing procedure of the control device 201. In the flowchart of FIG. 8, first, the control device 201 acquires the input data x to the learned model M (step S801). Next, the control device 201 inputs the acquired input data x to the encoder EC of the learned model M, and converts the input data x into the intermediate representation $H_0$ (step S802).

The control device 201 inputs the converted intermediate representation $H_0$ to the decoder DC of the learned model M, and generates the output data $y_0$ for the input data x (step S803). Next, with respect to the generated output data $y_0$, the control device 201 acquires a score $\phi_i(y_0)$ to be used for the energy function $E(y)$ (step S804).

Note that i is "i = 1, 2, ..., k" and k is "the number of types of score based on the learned model M". For example, in the case where the learned model M is "AlphaFold2", when k is "k = 2", ($\varphi_1(y_0)$ corresponds to $A_1(y_0)$ representing the confidence of an output structure, and $\varphi_2(y_0)$ corresponds to $A_2(y_0)$ representing the likeness to a target structure of protein.

In the case where the learned model M is the "Transformer model", when k is "k = 2", $\phi_1(y_0)$ corresponds to $A_1(y_0)$ representing an appearance probability of a sentence, and $\phi_2(y_0)$ corresponds to $A_2(y_0)$ indicating the degree of certainty of output data. $D_l(y, y^{target})$ may be included in the score $\varphi_i(y_t)$.

Next, the control device 201 calculates an energy value E(yt) for the current output data $y_t$ based on the score $\phi_i(y_t)$ using the energy function $E(y)$ of the above formula (1) (step S805). In the initial processing of step S805, an energy value $E(y_0)$ is calculated for the current output data $y_0$ based on the score $\varphi_i(y_0)$.

The control device 201 updates an intermediate representation $H_t$ to an intermediate representation $H_{t+1}$ by a predetermined search algorithm based on the calculated energy value $E(y_t)$ such that the energy function $E(y)$ is minimized (step S806). Next, the control device 201 inputs the updated intermediate representation $H_{t+1}$ to the decoder DC of the learned model M, and updates the output data yt to output data $y_{t+1}$ (step S807).

Next, the control device 201 acquires the score $\phi_i(y_t)$ to be used for the energy function $E(y)$ with respect to the updated output data $y_{t+1}$ (step S808). The control device 201 determines whether a predetermined end condition is satisfied (step S809). When the predetermined end condition is not satisfied (step S809: No), the control device 201 returns to step S805.

On the other hand, when the predetermined end condition is satisfied (step S809: Yes), the control device 201 outputs the updated output data $y_{t+1}$ (step S810), and ends the series of processing in this flowchart.

Consequently, the control device 201 may improve the quality of a generation result (output data $y_t$) acquired by manipulation of the intermediate representation $H_t$.

As described above, according to the control device 201 according to the embodiment, when the intermediate representation H of the input data x input to the learned model M is manipulated (changed), the validity of the current output data y generated from the intermediate representation H by the learned model M may be evaluated based on the first indicator, and the intermediate representation H may be updated (changed) based on the evaluation result such that the validity of the output data y generated by the learned model M increases. The first indicator is an indicator correlated with an existence probability of output data in the data distribution of learning data used for learning of the learned model M.

Consequently, the control device 201 may control manipulation of the intermediate representation H such that a valid output (output data y) is obtained. For example, by using the first indicator, the control device 201 may control manipulation of the intermediate representation H such that an output that is highly likely to exist in the real world is obtained. For this reason, the control device 201 may obtain a valid output more efficiently than in a case where random manipulation of the intermediate representation H is repeated.

According to the control device 201, the validity of the current output data y may be evaluated based on the first indicator and the second indicator representing the closeness to the target output data $y^{target}$.

Consequently, by using not only the first indicator but also the second indicator, the control device 201 may control manipulation of the intermediate representation H such that an output that is highly likely to exist in the real world and is close to data desired by a user is obtained.

According to the control device 201, a deep learning model that uses an amino acid sequence as input data and outputs output data representing a structure of protein (for example, AlphaFold2) may be used as the learned model M.

Consequently, the control device 201 may add a change to the intermediate representation H in a direction in which there is a high possibility that a valid structure of protein is obtained.

According to the control device 201, an indicator representing the confidence of an output structure estimated for the current output data y by the learned model M may be used as the first indicator. Note that this is the case where the learned model M is a deep learning model such as AlphaFold2.

Consequently, the control device 201 may control manipulation of the intermediate representation H such that a structure of protein that is highly likely to exist in the real world is obtained.

According to the control device 201, a score representing the likeness to a target structure of protein estimated for the structure of protein represented by the current output data y by the first classification model different from the learned model M, may be used as the second indicator. Note that this is the case where the learned model M is a deep learning model such as AlphaFold2.

Consequently, the control device 201 may control manipulation of the intermediate representation H such that an output close to a structure of protein desired by a user is obtained as a structure of protein that is highly likely to exist in the real world.

According to the control device 201, the degree of similarity between a known structure similar to the structure of protein represented by the initial output data $y_0$ generated from the initial intermediate representation $H_0$ converted from the input data x among the known structures of protein stored in the structure database (not illustrated) and the structure of protein represented by the current output data y, may be used as the second indicator. Note that this is the case where the learned model M is a deep learning model such as AlphaFold2.

Consequently, the control device 201 may control manipulation of the intermediate representation H such that an unknown structure is obtained as a structure of protein that is highly likely to exist in the real world.

According to the control device 201, the degree of similarity between the electron density distribution corresponding to a target structure of target protein and the electron density distribution corresponding to the structure of protein represented by the current output data y, may be used as the second indicator. Note that this is the case where the learned model M is a deep learning model such as AlphaFold2.

Consequently, the control device 201 may control manipulation of the intermediate representation H such that an output close to a structure of protein desired by a user is obtained when the electron density distribution corresponding to the target structure is known even if the specific target structure is not known.

According to the control device 201, a deep learning model that uses sequential information representing a sentence as input data and outputs sequential information representing another sentence as output data (for example, the Transformer model) may be used as the learned model M.

Consequently, the control device 201 may add a change to the intermediate representation H in a direction in which there is a high possibility that a valid sentence is obtained.

According to the control device 201, a score representing an output probability of a sentence estimated for the current output data y by the learned model M may be used as the first indicator. Note that this is the case where the learned model M is a deep learning model such as the Transformer model.

Consequently, the control device 201 may control manipulation of the intermediate representation H such that a sentence that is highly likely to exist in the real world is obtained.

According to the control device 201, a score indicating the degree of certainty of output data in the data distribution of the same learning data as that of the learned model M estimated for the current output data y by the second classification model learned by using the learning data, may be used as the first indicator. Note that this is the case where the learned model M is a deep learning model such as the Transformer model.

Consequently, the control device 201 may control manipulation of the intermediate representation H such that a sentence that is highly likely to exist in the real world is obtained.

According to the control device 201, a score representing the degree of conformance to an object label out of a plurality of labels classifying sentences estimated for a sentence represented by the current output data y by the third classification model different from the learned model M, may be used as the second indicator. Note that this is the case where the learned model M is a deep learning model such as the Transformer model.

Consequently, the control device 201 may control manipulation of the intermediate representation H such that an output close to a sentence having a nuance (for example, positive or negative) desired by a user is obtained as a sentence that is highly likely to exist in the real world.

According to the control device 201, an edit distance between a sentence represented by the initial output data $y_0$ generated from the initial intermediate representation $H_0$ converted from the input data x and a sentence represented by the current output data y, may be used as the second indicator. Note that this is the case where the learned model M is a deep learning model such as the Transformer model.

Consequently, the control device 201 may control manipulation of the intermediate representation H such that an output close to a different sentence that is not similar to the original sentence (output data $y_0$) is obtained as a sentence that is highly likely to exist in the real world.

According to the control device 201, whether a predetermined end condition is satisfied as a result of updating (changing) the intermediate representation H may be determined. According to the control device 201, when the predetermined end condition is not satisfied, the validity of the current output data y may be evaluated with the output data y generated from the updated intermediate representation H as the current output data y, and the intermediate representation H may be updated based on the evaluation result. According to the control device 201, when the predetermined end condition is satisfied, the output data y generated from the updated intermediate representation H may be output.

Consequently, the control device 201 may output a valid generation result (output data y) by repeating update of the intermediate representation H until the predetermined end condition is satisfied.

From the above, according to the control device 201, a change may be added to the intermediate representation H in a direction in which there is a high possibility that a valid output (output data y) is obtained, and a valid output may be obtained efficiently. For example, the control device 201 may obtain a valid output more efficiently than in the case where random manipulation is repeated for the intermediate representation H. The control device 201 may efficiently generate various high-quality outputs by generating new output data y for the same input data x by, for example, adding a change to the energy function E(y).

For example, the present control method may be applied to a structure prediction service that uses amino acid sequence information as input and outputs a structure of protein. In this case, according to the present control method, various high-quality structures of protein may be provided from a single amino acid sequence, and the quality of the structure prediction service may be improved.

For example, the present control method may be applied to a natural language processing service that uses sequential information representing a sentence as input and outputs another sentence. In this case, according to the present control method, various high-quality sentences may be provided from a single piece of sequential information (for example, a token ID string), and the quality of the natural language processing service may be improved.

The control method described in the present embodiment may be realized by executing a program prepared in advance by a computer such as a personal computer or a workstation. The present control program is recorded in a computer-readable recording medium such as a hard disk, a flexible disk, a CD-ROM, a DVD, or a USB memory, and is executed by being read from the recording medium by the computer. The present control program may be distributed via a network such as the Internet.

The information processing device 100 (control device 201) described in the present embodiment may be realized also by an application-specific integrated circuit (ASIC) such as a standard cell or a structured ASIC or a programmable logic device (PLD) such as a field-programmable gate array (FPGA).

The following appendices are further disclosed in relation to the above-described embodiment.

**Claims**

**1.** A control program for causing a computer to execute a process comprising:

when an intermediate representation of input data input to a learned model is changed,
evaluating validity of current output data generated from the intermediate representation by the learned model
based on a first indicator correlated with an existence probability of output data in a data distribution of learning data used for learning of the learned model; and

changing the intermediate representation based on an evaluation result such that validity of output data generated by the learned model increases.

2. The control program according to claim 1, wherein the evaluating includes

evaluating validity of the current output data based on the first indicator and a second indicator that represents closeness to target output data.

3. The control program according to claim 2, wherein

the learned model is a deep learning model that uses an amino acid sequence as input data and outputs output data that represents a structure of protein, and

the first indicator includes an indicator that represents confidence of an output structure estimated for the current output data by the learned model.

4. The control program according to claim 3, wherein the second indicator includes a score that represents likeness to a target structure of protein estimated for a structure of protein represented by the current output data by a first classification model different from the learned model.

5. The control program according to claim 3 or 4, wherein the second indicator includes a degree of similarity between a known structure similar to a structure of protein represented by initial output data generated from an initial intermediate representation converted from the input data among known structures of protein stored in a database and a structure of protein represented by the current output data.

6. The control program according to any one of claims 3 to 5, wherein the second indicator includes a degree of similarity between an electron density distribution that corresponds to a target structure of target protein and an electron density distribution that corresponds to a structure of protein represented by the current output data.

7. The control program according to claim 2, wherein

the learned model is a model that uses sequential information that represents a sentence as input data and outputs sequential information that represents another sentence as output data, and

the first indicator includes a score that represents an output probability of a sentence estimated for the current output data by the learned model.

8. The control program according to claim 7, wherein the first indicator includes a score that indicates a degree of certainty of output data in a data distribution of the learning data estimated for the current output data by a second classification model learned by using the learning data.

9. The control program according to claim 7 or 8, wherein the second indicator includes a score that represents a degree of conformance to an object label out of a plurality of labels that classifies sentences estimated for a sentence represented by the current output data by a third classification model different from the learned model.

10. The control program according to any one of claims 7 to 9, wherein the second indicator includes an edit distance between a sentence represented by initial output data generated from an initial intermediate representation converted from the input data and a sentence represented by the current output data.

11. The control program according to any one of claims 1 to 10, wherein the computer is caused to execute a process including

determining whether a predetermined end condition is satisfied as a result of changing the intermediate representation,

when the predetermined end condition is not satisfied, the evaluating and the changing, with output data generated from the changed intermediate representation set as the current output data, and

when the predetermined end condition is satisfied, outputting output data generated from the changed intermediate representation.

12. The control program according to claim 2, wherein

in the evaluating,
an energy value that corresponds to the current output data is calculated by using an energy function that includes a first term defined in a form in which the first indicator is included and a second term defined in a form in which the second indicator is included, and
in the changing,
the intermediate representation is changed based on the calculated energy value such that the energy function is minimized.

13. The control program according to any one of claims 1 to 12, wherein the learned model includes an encoder that converts the input data into an intermediate representation, and a decoder that refers to the intermediate representation and generates output data for the input data.

14. A control method implemented by a computer, the control method comprising:

when an intermediate representation of input data input to a learned model is changed,
evaluating validity of current output data generated from the intermediate representation by the learned model based on a first indicator correlated with an existence probability of output data in a data distribution of learning data used for learning of the learned model; and
changing the intermediate representation based on an evaluation result such that validity of output data generated by the learned model increases.

15. An information processing device comprising:

a control unit configured to
when an intermediate representation of input data input to a learned model is changed,
evaluate validity of current output data generated from the intermediate representation by the learned model based on a first indicator correlated with an existence probability of output data in a data distribution of learning data used for learning of the learned model, and
change the intermediate representation based on an evaluation result such that validity of output data generated by the learned model increases.

FIG. 1

# FIG. 2

Components:
- 201 (system)
- 304 DISK
- 303 DISK DRIVE
- 301 CPU
- 302 MEMORY
- 300 (bus)
- 305 COMMUNICATION I/F
- 306 GPU
- 307 PORTABLE TYPE RECORDING MEDIUM I/F
- 308 PORTABLE TYPE RECORDING MEDIUM
- 210 NETWORK

**FIG. 3**

# FIG. 4

INPUT DATA ~400

[ 0, 405, 128, 29, 10, 18452, 8, 747, 7920, 3251, 479, 2, 2]

# FIG. 5

# FIG. 6

# FIG. 7

# FIG. 8

```
        ┌──────────────┐
        │    START     │
        └──────┬───────┘
               │                        ⟋S801
    ┌──────────▼─────────────────┐
    │    ACQUIRE INPUT DATA x     │
    └──────────┬─────────────────┘
               │                        ⟋S802
    ┌──────────▼─────────────────┐
    │ INPUT TO ENCODER AND CONVERT INPUT │
    │    DATA x INTO INTERMEDIATE │
    │     REPRESENTATION H₀        │
    └──────────┬─────────────────┘
               │                        ⟋S803
    ┌──────────▼─────────────────┐
    │ INPUT TO DECODER AND GENERATE OUTPUT │
    │         DATA y₀             │
    └──────────┬─────────────────┘
               │                        ⟋S804
    ┌──────────▼─────────────────┐
    │   ACQUIRE SCORE Φᵢ(y₀)      │
    └──────────┬─────────────────┘
               │                        ⟋S805
    ┌──────────▼─────────────────┐
    │ CALCULATE ENERGY VALUE E(yₜ) FOR │
    │    CURRENT OUTPUT DATA y    │
    └──────────┬─────────────────┘
               │                        ⟋S806
    ┌──────────▼─────────────────┐
    │ UPDATE INTERMEDIATE REPRESENTATION Hₜ │
    │        Hₜ→Hₜ₊₁              │
    └──────────┬─────────────────┘
               │                        ⟋S807
    ┌──────────▼─────────────────┐
    │    UPDATE OUTPUT DATA yₜ    │
    │        yₜ→yₜ₊₁              │
    └──────────┬─────────────────┘
               │                        ⟋S808
    ┌──────────▼─────────────────┐
    │   ACQUIRE SCORE Φᵢ(yₜ)      │
    └──────────┬─────────────────┘
               │                        ⟋S809
          ◇───▼──────────◇        NO
         ◇ IS PREDETERMINED END ◇─────┐
         ◇ CONDITION SATISFIED? ◇     │
          ◇──────┬───────◇           │
                 │ YES               │
                 │            ⟋S810   │
    ┌────────────▼────────────┐      │
    │ OUTPUT OUTPUT DATA yₜ₊₁ │      │
    └────────────┬────────────┘
                 │
        ┌────────▼───────┐
        │     END        │
        └────────────────┘
```

Process flow (S801–S810):

- S801: ACQUIRE INPUT DATA x
- S802: INPUT TO ENCODER AND CONVERT INPUT DATA x INTO INTERMEDIATE REPRESENTATION $H_0$
- S803: INPUT TO DECODER AND GENERATE OUTPUT DATA $y_0$
- S804: ACQUIRE SCORE $\Phi_i(y_0)$
- S805: CALCULATE ENERGY VALUE $E(y_t)$ FOR CURRENT OUTPUT DATA y
- S806: UPDATE INTERMEDIATE REPRESENTATION $H_t$, $H_t \to H_{t+1}$
- S807: UPDATE OUTPUT DATA $y_t$, $y_t \to y_{t+1}$
- S808: ACQUIRE SCORE $\Phi_i(y_t)$
- S809: IS PREDETERMINED END CONDITION SATISFIED?
- S810: OUTPUT OUTPUT DATA $y_{t+1}$

Europäisches
Patentamt

European
Patent Office

Office européen
des brevets

**EUROPEAN SEARCH REPORT**

**Application Number**

EP 25 15 4886

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JUMPER JOHN ET AL: "Highly accurate protein structure prediction with AlphaFold", NATURE, SPRINGER NATURE LIMITED, LONDON, vol. 596, no. 7873, 15 July 2021 (2021-07-15), pages 583-589, XP037990370, ISSN: 0028-0836, DOI: 10.1038/S41586-021-03819-2 [retrieved on 2021-07-15] | 1-3,7-15 | INV. G16B40/20 G06N3/00 G16B15/20 |
| Y | * p. 583, left column p. 584, Figure 1e and description thereof p. 585, right column, par. 4 p. 590, left column, par. 4 p. 590, right column, par. 2 * & Jumper John ET AL: "Highly accurate protein structure prediction with AlphaFold - Supplementary Information", Nature, vol. 596, no. 7873, 15 July 2021 (2021-07-15), pages 583-589, XP055888904, DOI: 10.1038/s41586-021-03819-2 Retrieved from the Internet: URL:https://www.nature.com/articles/s41586-021-03819-2> * p. 11, section "1.4 AlphaFold Inference" p. 13, Algorithm 2 p. 33, section "1.9 Loss functions and auxiliary heads" p. 38, section "1.9.6 Model confidence prediction (pLDDT)" * | 4-6 | |

-----

-/--

**TECHNICAL FIELDS SEARCHED (IPC)**

G16B
G06N

The present search report has been drawn up for all claims

| Place of search | Date of completion of the search | Examiner |
|---|---|---|
| Munich | 8 July 2025 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 25 15 4886

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RONEY JAMES P. ET AL: "State-of-the-Art Estimation of Protein Model Accuracy Using AlphaFold", PHYSICAL REVIEW LETTERS, vol. 129, no. 23, 28 November 2022 (2022-11-28), pages 1-6, XP093293820, US ISSN: 0031-9007, DOI: 10.1103/PhysRevLett.129.238101 Retrieved from the Internet: URL:https://journals.aps.org/prl/abstract/10.1103/PhysRevLett.129.238101> | 4,5 | |
| A | * p. 2, right column, par. 3 p. 5, left column, par. 1 * | 1-3,6-15 | |
| Y | TERASHI GENKI ET AL: "Protein model refinement for cryo-EM maps using AlphaFold 2 and the DAQ score", ACTA CRYSTALLOGRAPHICA / D. SECTION D, BIOLOGICAL CRYSTALLOGRAPHY, vol. 79, no. 1, 1 January 2023 (2023-01-01), pages 10-21, XP093293460, Oxford ISSN: 2059-7983, DOI: 10.1107/S2059798322011676 Retrieved from the Internet: URL:https://journals.iucr.org/d/issues/2023/01/00/ji5028/index.html> | 6 | |
| A | * the whole document, in particular teh Abstract * | 1-5,7-15 | TECHNICAL FIELDS SEARCHED (IPC) |

The present search report has been drawn up for all claims

| Place of search | Date of completion of the search | Examiner |
|---|---|---|
| Munich | 8 July 2025 | Mühlbauer, Max |

## EP 4 621 789 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VASWANI et al.** Attention is All You Need. *NeurIPS 2017*, 2017
- **JUMPER, J. et al.** Highly accurate protein structure prediction with AlphaFold.. *Nature*, 2021, vol. 596, 583-589
- **VASWANI et al.** attention is All You Need. *NeurIPS 2017*, 2017
- **RADFORD et al.** *Language Models are Unsupervised Multitask Learners*, 2019